(19) Europäisches Patentamt
European Patent Office
Office européen des brevets

(11) **EP 2 958 593 B1**

(12) **EUROPEAN PATENT SPECIFICATION**

(45) Date of publication and mention of the grant of the patent:
**12.05.2021 Bulletin 2021/19**

(21) Application number: **14754253.4**

(22) Date of filing: **21.02.2014**

(51) Int Cl.:
*A61K 47/10* (2017.01)     *A61K 31/5513* (2006.01)
*A61K 9/107* (2006.01)     *A61P 25/00* (2006.01)
*A61P 25/08* (2006.01)     *A61P 25/22* (2006.01)

(86) International application number:
**PCT/CA2014/000126**

(87) International publication number:
**WO 2014/127458 (28.08.2014 Gazette 2014/35)**

(54) **PHARMACEUTICAL COMPOSITION FOR TRANSMUCOSAL ADMINISTRATION OF LORAZEPAM**

PHARMAZEUTISCHE ZUSAMMENSETZUNG ZUR TRANSMUKOSALEN VERABREICHUNG VON LORAZEPAM

COMPOSITION PHARMACEUTIQUE POUR L'ADMINISTRATION TRANSMUQUEUSE DE LORAZEPAM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.02.2013 US 201361767898 P**

(43) Date of publication of application:
**30.12.2015 Bulletin 2015/53**

(73) Proprietor: **Eastgate Pharmaceuticals Inc.
North York, Ontario M3J 2T9 (CA)**

(72) Inventors:
• **SCHWARZ, Joseph**
  **Toronto, Ontario M2M 4B9 (CA)**
• **WEISSPAPIR, Michael**
  **Toronto, Ontario M3H 4P1 (CA)**
• **CARLEN, Peter Louis**
  **Toronto, Ontario M3H 3V9 (CA)**

(74) Representative: **August Debouzy**
**7, rue de Téhéran**
**75008 Paris (FR)**

(56) References cited:
WO-A1-03/004015     WO-A1-2005/032517
WO-A1-2008/075102     WO-A1-2009/021106
WO-A2-2011/135314     CA-A1- 2 379 365
CA-A1- 2 529 489     US-A1- 2001 055 571

• A Chauvet ' ET AL: "ETUDE THERMOANALYTIQUE ET SP~E DE L'INrERACTION LORAZEPAM/PEG 6000", Journal of Thermal Analysis, 1 January 1995 (1995-01-01), pages 473-487, XP055283693, Retrieved from the Internet: URL:http://rd.springer.com/content/pdf/10.1007/BF02636137.pdf

**Description**

BACKGROUND OF INVENTION

The benzodiazepines are the one of the most prescribed pharmacological class over the last four decades. Practically all clinically important effects of the benzodiazepines are result of their actions on the CNS. This class has a broad spectrum of clinical uses. Among different successful clinical applications the most important are: convulsive diseases, anxiety, sleep and mood disorders, panic attacks, psychiatric diseases, treatment of alcohol and narcotic withdrawal, premedication before various diagnostic and surgical interventions and many other indications. All the benzodiazepines have similar pharmacological profiles but differ in selectivity, doses and pharmacokinetic parameters. Furthermore the benzodiazepines are characterized by favorable safety profile.

Acute seizures, e.g., Status epilepticus (SE) and cluster seizures are common and potentially life-threatening neurologic emergency characterized by prolonged seizures. The reported annual frequency of SE cases in the United States has been between 102,000 and 152,000, with roughly 55,000 of these incidents proving fatal. Since the estimated mortality range from 17% to 23% and morbidity from 10% to 23%, the impact of SE and cluster seizures are dramatic [Behrouz et al., 2009]. Current annual costs in US exceed $4 billion to identify and treat cases with subsequent hospitalization [Tatum et al., 2001].

The emergency medical community is striving to improve the care for patients suffering from seizures by examining the drug-delivery techniques to reduce the time to treatment and cessation of seizures. Antiepileptic drugs are commonly given orally for chronic treatment of epilepsy. The treatment of epilepsy requires different types of medications for both acute and chronic phases of the disease. Parenteral routes of administration of antiepileptic drugs usually employed when a rapid clinical response is required. There is a significant need for new dosage forms of the antiepileptic drugs for emergency situations when use of the traditional parenteral dosage forms are not possible especially in out of hospital settings. [Anderson et al., 2012].

The main target for treatment of acute epileptic seizures is termination the attacks as soon as possible. The longer epileptic seizures exists the harder to control them with anticonvulsant drugs and the risk of permanent brain damage increases. Therefore, it is very important to promptly treat patients by administering to them an adequate dose of an effective anticonvulsant medicine. Diazepam, Midazolam and Lorazepam are benzodiazepines that have been most widely used for this purpose. The intravenous administration of an anti-convulsant is the fastest way to suppress epileptic seizures. However, intravenous administration during a seizure attack is not easy and usually can be provided in hospitals. Obviously it is a serious demand in new non-parenteral dosage forms of anticonvulsants. Additionally, fast working transmucosal formulations of benzodiazepines could be useful for prevention and treatment of panic attacks, panic disorder, phobias, psychiatric disorders, excitation and insomnia as well as premedication and other conditions.

BACKGROUND OF THE INVENTION

One of the extensively investigated alternatives of parenteral delivery of anticonvulsants is intranasal administration. The broad spectrum of different intranasal formulations containing benzodiazepines has been investigated in the last decade. Nasal mucosa is highly vascularized and provides a virtual route for penetration of many medicinal substances. The nasal administration has various advantages in term of convenience of administration to achieve systemic or topical effects. However, intranasal route typically suitable for water soluble drugs, but many benzodiazepines have very limited solubility in water. The leading problem associated with the nasal administration of drugs is the limited volume of administration. In general, it is impossible to administer drugs in a volume more than 150 mcl per a nostril because the bigger volume will be swallowed [Wermeling, 2009]. An additional restriction for the nasal delivery is sensitivity of the nasal mucosa to the irritation potential of different drugs and excipients.

As disclosed in US patent application 20110172211 an intranasal anticonvulsive pharmaceutical compositions includes a poorly soluble anti-convulsant. The anticonvulsive pharmaceutical composition comprising a poorly soluble anticonvulsant as an active component, which is intranasally spray-administered, also comprises diethylene glycol monoethyl ether and fatty acid ester, wherein the fatty acid ester is selected from the group consisting of caprylocaproyl polyoxylglyceride, isopropyl palmitate, oleoyl polyoxylglyceride, Sorbitan monolaurate 20, methyl laurate, ethyl laurate, and polysorbate 20. Also, the anticonvulsive pharmaceutical composition comprising a poorly soluble anticonvulsant as an active component, which is intranasally spray-administered, comprises diethylene glycol monoethyl ether, fatty acid ester, methylpyrrolidone, water and alcohol. Therefore, the intranasal anticonvulsive pharmaceutical composition may be useful to enhance the bioavailability of the poorly soluble anticonvulsant. Additionally, the described intranasal anticonvulsive pharmaceutical composition may be useful for poorly soluble anticonvulsants in terms of the improved viscosity and/or enhanced solubility in order to effectively deliver the poorly soluble anticonvulsants in therapeutic doses. [Baek; Myoung-Ki; et al., US Patent Application 20110172211].

Benzodiazepines such as diazepam and lorazepam are difficult to develop into a formulation suitable for transmucosal administration since they have extremely low solubility in water and precipitate when dissolved in commonly used polar water miscible solvents such as propylene glycol, alcohol, PEG or dimethyl sulfoxide (DMSO) after contact with water media. Therefore, the development of solvent systems suitable for transmucosal administration, which dissolve a benzodiazepine (e.g. Diazepam, Lorazepam and some other) in a required concentration and prevent drug precipitation upon contact with a water media, is highly required.

The intranasal absorption of drugs may be augmented by administering a drug with a chemical aid or a penetration enhancer at the same time. For example, Lau and Slattery [1989] made an attempt to dissolve a benzodiazepine (i.e. diazepam) in various solvents (for example, triacetin, dimethylsulfoxide, PEG 400, Cremophor EL, Lipal-9-LA, diisopropyl adipate and azone). Despite the fact that diazepam can be dissolved in most of the solvents within a desired concentration, the use of these formulations are impractical due to high irritation potential of the solvents for nasal mucosae. Cremophor® EL has been found to have the lowest stimulus to the nasal mucosal tissue, but its nasal absorption is rather slow ($T_{max}$: 1.4 hours) in humans in use of these vehicles, and the peak concentration is lower than that observed after the intravenous administration.

Li, et al. [2002] proposed a microemulsion for rapid-onset intranasal delivery of diazepam. U.S. Patent No. 6,627,211 B1 discloses intranasal anticonvulsive compositions comprising diazepam, dissolved in a solubilizing vehicle, containing aliphatic alcohol, another polar solvent such as propylene glycol, water, etc.

US Patent Application 2005/0002987 A1 discloses a composition for intranasal administration used microemulsions containing diazepam. The diazepam is dissolved in a vehicle comprising equivalent amounts of fatty acid ester and water and the balance of hydrophilic surfactant, polar solvent (i.e. glycol), etc.

Intranasal administration of solvent based injectable compositions of Diazepam, Lorazepam and Midazolam showed anticonvulsant efficacy but is limited due to slow absorption, irritability and some difficulties in drug administration process due to limited volume of composition, which can be delivered into nostrils.

Unfortunately, all these approaches are not suitable for Lorazepam, one of the most potent and fast acting benzodiazepine anticonvulsant, due to two main obstacles: low solubility of Lorazepam in hydrophobic phases of microemulsions and extremely low stability of dissolved Lorazepam in presence of even low concentrations of water. Optimal amount of liquid to be administered and absorbed directly through intra-oral, sublingual or buccal mucosal lining is approximately 100-500 mcl, excess could be swallowed and absorption will be significantly delayed. Since the usually recommended doses of Lorazepam are approximately 2-4 mg, the concentration of the drug in a transmucosal composition should be about 10 mg/mL. This concentration can be reached using polar solvents, such as alcohol, propylene glycol, DMSO, N-Methylpyrrolidone, liquid PEG or Transcutol® (Monoethyl ether of Diethyleneglycol) however these solvents can cause serious irritation of oral mucosae. Moreover, contact such solutions with saliva (water media) usually causes drug crystallization and precipitation. Additionally, high hygroscopicity of polar solvents causes decreased stability of Lorazepam in solutions. Incorporation of short triglycerides, such as Triacetin, into vehicle, improves stability but delays onset of anticonvulsant action. Triglycerides of higher fatty acids demonstrate low solubility of Lorazepam and cannot keep the desired concentration of the drug in dissolved stage.

Intranasal delivery was found feasible for stable benzodiazepines, namely Diazepam, Clonazepam and Midazolam. The last one, due to good water solubility, provides noticeable anti-seizure activity delivered into human patients with onset of action in approximately 10 minutes. Nevertheless, the use of such products is limited by irritation of nasal mucosa and difficulties in administering of viscous formulations. [Clonazepam spray described in US Patent Application 20080070904; Intranasal benzodiazepine spray disclosed in US Patent Application 20110172211A1]

In some cases use of buccal administration of water soluble benzodiazepine, Midazolam, was found effective in the treatment of acute seizures. Buccolam® (Midazolam oromucosal solution) has recently acquired a pediatric use marketing authorization from the European Commission to become the first and only licensed oromucosal Midazolam for the treatment of prolonged, acute, convulsive seizures in infants, children and adolescents (from 3 months to <18 years of age). Following buccal administration, it is rapidly absorbed across the mucous membranes directly into the bloodstream. Clinical studies show that cessation of visible signs of seizures within ten minutes was achieved in 65-78% of children receiving oromucosal Midazolam [Jevon, 2012]. Buccolam® has limited shelf life (18 months) and onset of action is still slower than after parenteral administration.

Possible approaches for intraoral formulations of Lorazepam, such as submicron oil-in-water emulsions, phospholipid-bile salt mixed micelles or micellar solutions with high concentrations of surfactants, investigated by Giovannone [WO 2004/004783 A1], cannot provide a stable products for transmucosal drug administration. For example, a composition based on combination of polar solvents (Transcutol, PEG or PG) and acetyl triglyceride (Triacetin) has short shelf life and the dissolved drug (2 mg/mL) should be delivered in relatively large volumes and easily precipitates after contact with water media. Solubility of Lorazepam in oils is much lower than solubility in polar solvents. A combination of an oil and polar solvents is not efficient since after contact with water media polar solvent diffuses to water, forcing drug crystallization in oil and precipitation from water phase.

Mixed micelles and phospholipid-cholate aggregates, tested by Hammad [1999] also does not allow to reach desired solubility Coghill describes a powder formulation of lorazepam for buccal delivery [WO2011135314].

For effective suppression of seizures in patients an estimated Lorazepam doses usually between 1 to 4 mg. Additionally, the optimal volume of a liquid transmucosal formulation which could be delivered sublingually, is about 100-500 mcl, excess could be swallowed. For swallowed portion of a composition drug delivery to the blood and subsequently to the brain will be postponed for 1-2 hours.

Due to abovementioned, the required concentration of Lorazepam must be around 10 mg/mL. It could be easily achieved when polar solvents (PEG, Propylene glycol, Benzyl alcohol) are used. For oil-in-water emulsions where oil phase is no more than 20-30% of the composition, required solubility of Lorazepam in the oil phase should be approximately 30-50 mg/ml.

DESCRIPTION OF THE INVENTION

It is obvious that intraoral transmucosal administering of a anticonvulsant drug such as Lorazepam to the patient in need is more practical and convenient than parenteral, intranasal or rectal routes. This can be reached by use of liquid compositions, preferably sprayable, which can be delivered sublingually or onto mucosal surface of gums, palate or cheeks and lips, especially to the unconscious patients or during the ongoing seizures. Transmucosal liquid compositions should have relatively low viscosity to provide possibility of fast delivery, and must provide fast onset of anticonvulsive action.

Extremely low water solubility of Lorazepam (~27 mcg/ml in saline) causes precipitation of the drug form injectable solution (Ativan®) in mixture of propylene glycol (80%), polyethylene glycol 400 (18%) and benzyl alcohol (2%) upon dilution with water in ratio 1:5 and higher. Intraoral administration of the injection solution (Ativan®) to mice demonstrated lack of anti-seizure activity while intraperitoneal delivery provides distinct protection even 2-3 minutes after injection (see Graph 1).

Low solubility of Lorazepam limits possibility of incorporation the required amount of the drug into oil-in-water emulsions.

It was surprisingly found that Lorazepam forms low melting eutectic mixtures with some organic molecules, and these eutectic mixtures possess much higher solubility in oils than pure Lorazepam. Eutectic mixtures with seriously enhanced oil solubility were obtained when Lorazepam was combined with menthol (L-Menthol), phenol, chlorobutanol, thymol, and some other molecules.

The most pronounced increase in solubility was found for L-Menthol and for Thymol. Solubility at room temperature in different triglyceride oils increased almost 10 times for eutectic mixture Lorazepam : L-Menthol (melting point 36-37°C) and about one order of magnitude for Lorazepam : Thymol. Menthol or Thymol may be used either as crystalline purified materials or as components of naturally available essential oils, such as peppermint oil, spearmint oil, thyme oil, oregano oil and basil oil and some others.

Additionally it was found that incorporation of eutectic mixture of Lorazepam apparently improves easiness of emulsification and results in smooth self-emulsifying compositions with high concentration of the drug. Moreover these formulations require reasonably low concentrations of surfactants and thus decreasing irritation potential of such formulations.

The proposed Lorazepam composition for intraoral administration according to the invention is prepared by dissolving Lorazepam in combination of eutectic component(s) and hydrophobic (oil) phase, containing one or several surfactants. An oil phase comprises of a glyceride oil, aliphatic or aromatic esters, and tocopherols and tocopheryl esters or an appropriate mixture of these hydrophobic components.

Prepared compositions, containing Lorazepam, can be administered using dropper, oral syringe or hand pump.

Finally, the composition of invention may be delivered to patient in need intraorally by administering sublingually (under the tongue), buccally (on the cheek mucosa), on gums or lips or any area of internal surface of the mouth. This is extremely important for treatment of unconscious patient or during the seizures.

The proposed composition is not irritating and provides fast onset of the anti-seizure action, comparable with parenteral administration of Lorazepam. The dose is delivered in the form of droplets; This composition can be easily self-administered. Beside treatment of acute seizures, proposed composition can be used for tranquilizing, sedation, premedication for kids and adults, prevention and treatment of panic attacks or sleep disorders.

ANTI-SEIZURE ACTIVITY EVALUATION

For investigation of anti-seizure activity of invented formulations, a timed intravenous PTZ infusion test was employed.

Timed intravenous PTZ infusion test

The technique of timed i.v. PTZ (pentylenetetrazole) infusion test (ivPTZ) is a simple, reproducible and fast method for assessment of onset, peak, duration and potency of various anticonvulsants and proconvulsants in animals. This test provides an extremely sensitive parametric method for assessing seizure threshold in individual animals and a quantifiable endpoint can be obtained with a minimal number of animals. Different seizure types can be chosen as endpoint in this test. Usually, the first seizure occurring during the PTZ infusion is a myoclonic twitch, followed by clonic and, later, tonic seizures.

This method offers many advantages over the more widely used classical subcutaneous PTZ (scPTZ) test. Since with ivPTZ, test effects based on different endpoints are measured in individual animals, the number of animals required for a proper evaluation is much lower than would be needed for standard scPTZ test. Additionally, significantly lower variability is observed during the ivPTZ technique than during other methods since each animal is used as its own control. The ivPTZ procedure is based on previously described methods (Giardina and Gasior, 2009; Mandhane et al., 2007;).

Method description

A butterfly catheter (Small animal butterfly catheter infusion set, needle size 27 G, 3/8 in., Harvard Apparatus, St. Laurent, Quebec, Canada) attached to a 5 ml syringe prefilled with heparinized 1% PTZ solution is used. The animal is restrained (Nose Cone Animal Holder, Kent Scientific, Torrington, Connecticut) and the needle is inserted into the tail vein. The accuracy of needle placement into the vein is confirmed by the appearance of blood in the cannula. The needle is secured to the tail by a plastic tape. The animal is transferred to a transparent plastic box and kept there for the duration of the test that takes only few minutes. The syringe is held in a syringe infusion pump (Syringe pump 22, Harvard Apparatus, St. Laurent, Quebec, Canada) which provides a constant speed infusion (0.3 ml/min) of the PTZ solution. PTZ sterile solution is continuously infused via a thin, very flexible plastic catheter permanently connected to the small gauge (27 G) short needle. This procedure allows animals to move freely in the box without noticeable pain or distress for duration of PTZ infusion (2 minutes).

During the infusion, mice are observed for the onset of different types of seizures. The time latencies from the start of infusion to the appearance of the first clonus (characterized by rapid involuntary rhythmic contraction and relaxation of limbs), lasting longer than 5 seconds, are recorded. Infusion is immediately stopped at the appearance of this endpoint. Forelimb clonus is the more reliable endpoint with less variation than other types of seizures for the ivPTZ test. The endpoint for the "clonic latent period" was noted at the moment the mouse fell on its side and showed a jerking of the head and forelimbs.

The infusion of PTZ will be terminated at 2 min, in case of non-appearance of clonic seizures. For such animals, the dose of PTZ in mg/kg infused during the course of 2 min is calculated as the threshold dose. **The threshold doses** of PTZ producing clonic seizures are calculated (in mg/kg) using the following formula:

$$\frac{Vinf * Tons * Cptz * 1000}{60 * Bweight}$$

where:

**Vinf** - rate of infusion (ml/min),
**Tons** - time for onset of seizure (sec),
**Cptz** - concentration of PTZ (mg/ml),
**Bweight** - body weight of animal (g).

The higher is threshold level, the more pronounced is anti-seizure activity of the tested composition. E.g., PTZ dose threshold for saline or vehicle (5 minutes time lap) value is about 50 mg/kg (for selected rate of infusion 0.3 ml/min and PTZ concentration 10 mg/ml) while for intraperitoneally administered Lorazepam 2.5 mg/kg (positive control) it reaches -100-120 mg/kg (at same 5 minutes time point).

FORMULATIONS

EXAMPLE 1.

Preparation of injectable Lorazepam solution (comparative solution)

Lorazepam was dissolved in a solvent vehicle, containing propylene glycol (80%), polyethylene glycol 400 (18%) and benzyl alcohol (2%), forming clear solution with drug concentration 2 mg/ml. Prepared solution was stored refrigerated and used in 30 days from the preparation date.

Example 2

Lorazepam Eutectic mixtures are presented in table 1.
After combining of pre-weighed amounts of components they were gently triturated at room temperature (20-22°C) using a stainless steel spatula until liquid phase is formed.

Table 1. Lorazepam Eutectic mixtures

| Compound | Melting point | Molecular weight | Solubility in capric/caprylic triglycerides (MCT oil) |
|---|---|---|---|
| L-Menthol (2-isopropyl-5-methylcyclohexanol) | 41-43°C | 156.21 | |
| Phenol | 38.5 -40.5°C | 94.11 | |
| Thymol (2-Isopropyl-5-methylphenol) | 49-51°C | 150.22 | |
| Lorazepam (99.8%) | 166-168°C | 321.16 | 4.3 mg/ml |
| 1 part Lorazepam + 2.5 parts L-Menthol | 36-39°C | N/A | 10.8 mg/ml |
| 1 part Lorazepam + 10 parts L-Menthol | 36-39°C | N/A | 40 mg/ml |
| 1 part of Lorazepam + 4.5 parts of phenol | <20°C | N/A | 38 mg/ml |
| 1 part of Lorazepam + 3 parts of thymol | 32-35°C | N/A | 35 mg/ml |

The data in Table 1 is presented for illustration purposes only. Other compounds may also form physiologically acceptable eutectic mixtures with Lorazepam, having lower melting points and better oil solubility than individual components.
Addition of menthol to other vehicle significantly increases Lorazepam solubility. In pure capric/caprylic triglycerides (MCT oil) solubility at RT is 4.3 mg/ml. Addition of 5 % Menthol by weight increases solubility by 28%, to 5.5 mg/ml, MCT with 8% Menthol in oil dissolves 6.8 mg/ml of the drug (~ 60% increase). Solubility of Lorazepam in peppermint oil, containing about 40% menthol, reaches 38.9 mg/ml.

SELF-EMULSIFYING COMPOSITIONS

Self-emulsifying drug delivery systems (SEDDS) are well known, but each composition of self-emulsifying drug delivery system must be developed separately for combination of active compound and selected excipients since even minor variations in composition requires careful choice of components such as surfactants, co-surfactants, oils, solvents their weigh relations and preparation process.
As surfactants and oil phase constituents, the pharmaceutical or food grade compounds were selected to assure safety and physiological compliance of the prepared formulations.
Some of investigated compositions are presented below.
Comparative example: Lorazepam injectable solution (Reference formulation).
Vehicle contains 80% Propylene glycol, 18% Polyethylene glycol 400 and 2% Benzyl alcohol; Lorazepam (2 mg/mL) was dissolved in the vehicle and stored tightly closed in a refrigerator.
**Example 1.** Peppermint oil based micelle-forming solution.
Vehicle contains 2% of peppermint oil (NF grade), 2% Polysorbate-20 (Tween®-20, NF) and 96% of Di(ethylene glycol) monoethyl ether (Transcutol®, EP). Lorazepam (10 mg/mL) was dissolved in the vehicle and stored tightly closed in a refrigerator.
**Example 2.** Nanoemulsion forming composition with crystalline L-Menthol.
Vehicle was prepared by dissolving crystalline L-Menthol (3%) and acetylated monoglyrerides (42%), containing lecithin and alcohol (6.5% of each), D- alpha-Tocopherol (2%), Polysorbate-20 (18%) and polyethoxylated hydrogenated castor oil (22%). Lorazepam (10 mg/mL) was dissolved in the vehicle at 40-45°C, and prepared solution was stored tightly closed in refrigerator.
**Example 3.** Nanoemulsion forming composition without Menthol.
Vehicle was prepared by mixing acetylated monoglyrerides (45%), lecithin and alcohol (6.5% of each), D-alpha-Tocopherol (2%), Polysorbate-20 (18%) and polyethoxylated hydrogenated castor oil (22%). Lorazepam (10 mg/mL) was dissolved in the vehicle at 40-45°C, and prepared solution was stored tightly closed in refrigerator.

Other vehicles for transmucosal intraoral delivery of Lorazepam were prepared in similar manner, using dry and anhydrous components (see tables 3-7). Behavior of the composition on contact with water was examined using artificial saliva. In most cases after mixing with water media a stable micellar solution, emulsion or submicron emulsion (nanoemulsion) were immediately formed, depending on used constituents. Nevertheless, in some cases formed colloidal dispersions were coarse and unstable, followed by visible phase separation.

Table 3. Self-nanoemulsifying and micelle forming compositions for Lorazepam (examples 4-14)

| Example No. | 4 | 5 | 6 | 7 | 8 | 9 | 10 | 11 | 12 | 13 | 14 |
|---|---|---|---|---|---|---|---|---|---|---|---|
| Peppermint oil | 6.5% | 6.0% | 5.5% | 6.3% | 5.7% | 5.0% | 3.1% | 3.7% | 5.0% | 4.5% | 4.0% |
| Lecithin | 27.1% | 25.1% | 23.1% | 12.6% | 11.4% | 6,3% | 6.2% | 5.5% | 2.4% | 2.7% | 3.5% |
| Polyethoxylated hydrogenated castor oil (Cremophor® RH-40) | 13.0% | 12.0% | | | | | 14.9% | | | | 15.0% |
| PEG-20 Sorbitan monooleate (Polysorbate-80) | | | 12.9% | 20.2% | 18.3% | 20.0% | | | | | |
| PEG-20 Sorbitan monostearate (Polysorbate-60) | | | | | 9.2% | | | 10.1% | 12.4% | 6.3% | 15.0% |
| PEG-20 Sorbitan monolaurate (Polysorbate-20) | | | | | | | | | | 16.2% | |
| d-alpha-Ttocopheryl polyethylene glycol 1000 succinate (TPGS) | | 7.2% | | | | | 8.3% | | | | |
| Caprylocaproyl polyoxyl-8 glycerides (Labrasol®) | | | | | | | | 18.3% | | | |
| Acetylated monoglycerides | | | | | | | | | | 25.9% | 23.0% |
| Capric/caprylic triglycerides | | | 16.6% | 22.7% | 20.6% | 25.0% | 20.7% | 20.2% | 29.7% | | |
| Tocopherol acetate | 4.3% | 4.0% | | | | | 9.3% | 9.2% | 9.9% | 5.6% | |
| Ethanol | 48.6% | 45.2% | 49.6% | 37.8% | 47.0% | 31.2% | 37.3% | 33.0% | 39.6% | 38.7% | 39.5% |
| Citric acid | 0.4% | 0.4% | 0.4% | 0.5% | 0.5% | | | | | | |
| Appearance after addition of 0.1 ml to 0.5 ml of artificial saliva (at 37°C) | -- | +++ | + | ++ | +++ | +- | + | + | + | ++ | +++ |

EP 2 958 593 B1

Table 4. Self-nanoemulsifying vehicle compositions for Lorazepam (examples 15-25)

| Example No. | 15 | 16 | 17 | 18 | 19 | 20 | 21 | 22 | 23 | 24 | 25 |
|---|---|---|---|---|---|---|---|---|---|---|---|
| Peppermint oil | 3.0% | 3.0% | 4.9% | 5.5% | 5.5% | 3.0% | 3.0% | 4.3% | 4.2% | 3.9% | 4.1% |
| Lecithin | 3.4% | 3.4% | 5.7% | 6.4% | 6.4% | 2.5% | 3.0% | 3.8% | 3.7% | 3.4% | 3.6% |
| Polyethoxylated hydrogenated castor oil (Cremophor RH-40) | 14.9% | 14.8% | 24.3% | 21.8% | 21.3% | 13.1% | 0.0% | 16.2% | 15.6% | 9.7% | 10.2% |
| PEG-20 Sorbitan monooleate (Polysorbate-80) | | | | | | | 12.0% | | | | |
| PEG-20 Sorbitan monostearate (Polysorbate-60) | 14.9% | 14.8% | 8.1% | | | | | 16.2% | 15.6% | | |
| PEG-20 Sorbitan monolaurate (Polysorbate-20) | | | | 18.2% | 18.2% | | | | | | |
| Caprylocaproyl polyoxyl-8 glycerides (Labrasol®) | | | | | | 18.7% | | | | 9.7% | 10.2% |
| Dimyristoylphosphatidylglycerol sodium salt (DMPG Na) | 0.4% | 0.4% | | | | 0.9% | 1.0% | | 0.3% | 0.4% | 0.4% |
| Distearoylphosphatidylglycerol sodium salt (DSPG Na) | | | | | 0.5% | | | | | | |
| Acetylated monoglycerides | 22.8% | 22.6% | 48.5% | 40.0% | 40.0% | 26.2% | | 14.0% | 15.6% | 14.5% | 10.2% |
| Acetyltributylcitrate | | | | | | | 30.0% | | | | |
| Tocopherol acetate | | | | | | | | | | | |
| Mixed Tocopherols | 1.4% | 1.8% | 2.9% | 1.8% | 1.8% | | | 2.6% | 4.2% | 1.9% | 2.0% |
| Oleic acid | | | | | | 10.0% | | | | | |
| Sodium lauryl sulfate (SLS) | | | | | | | | 0.3% | | | |
| Sodium Desoxycholate | | | | | | 4.0% | | | | | |
| Ethanol | 39.1% | 38.9% | 5.6% | 6.3% | 6.3% | 35.5% | 37.0% | 21.1% | 20.2% | 27.6% | 23.9% |
| Propylene glycol | | | | | | | | 21.6% | 10.4% | 29.0% | 25.4% |
| Triacetin (Captex 500) | | | | | | | | | 10.4% | | 10.2% |
| Sucralose | | 0.4% | | | | | | | | | |
| Appearance after addition of 0.1 ml to 0.5 ml of artificial saliva (37°C) | ++++ | ++++ | +- | ++ | ++ | + | + | ++++ | ++++ | ++++ | ++ |

EP 2 958 593 B1

## EXPERIMENTAL ANTI-SEIZURES ACTIVITY OF LORAZEPAM FORMULATIONS

Several prepared formulations of Lorazepam were tested for seizure protection properties (ivPTZ test). A marketed solution of Lorazepam in water miscible combination of Polyethylene glycol, Propylene glycol and benzyl alcohol was used as a comparator. Intraperitoneal administration of Lorazepam solution in a dose of 2.5 mg/kg leads to pronounced increase of PTZ threshold, confirming a prominent anti-seizure activity of Lorazepam, while the same formulations, administered intraorally on the mucosal surface of the mouth demonstrated total lack of such activity (Graph 1).

Incorporation of Lorazepam in formulations, that have ability to prevent precipitation of the benzodiazepine after contact with water media, may improve anti-seizure activity of Lorazepam. For example, addition of Menthol, increasing solubility of the drug in the oil phase causes significant improvement of anticonvulsive properties, as shown in Graph 2.

The onset of anticonvulsant activity is one of the most important parameters for a transmucosal anti-seizure formulation. For some formulations a high level of protection were observed even in 2 minutes after delivery of a formulation, in other compositions onset could be delayed, but at 5 minutes it was clearly developed, as shown in Graph 3.

Formulations, containing no organic solvents, have high viscosity and, despite fast onset of action are not convenient for delivery since viscous materials (viscosity > 50-100 cP) are difficult to spray. Use of nonvolatile solvents, such as polyethylene glycols, propylene glycol, Transcutol® and some other, led to relatively viscous products (viscosity > 50 cP) with mediocre anti-seizure activity.

Dilution of compositions with volatile Ethyl alcohol provides easily sprayable compositions with low viscosity (e.g., Lorazepam was dissolved in mixture of 8 parts of Example 18 and 2 parts of anhydrous alcohol).

Ant-seizure action of different tested Lorazepam formulations develops gradually. For the parenterally administered Lorazepam maximal protection observed at approximately 5 minutes, protection after delivery of intraoral transmucosal formulations increases steadily to 20 minutes, while effective level of protection is reached after 5 minutes.

On Graph 4 presented median particle size and size distribution of the oil droplets for a nanoemulsion, formed from formulation of Example 18, diluted with artificial saliva. This nanoemulsion has mean size around 40 nm and a narrow (PDI<0.2) size distribution pattern.

CONCLUSIONS

Lorazepam formulations, based on eutectic mixtures of a benzodiazepine with Menthol or Thymol and forming nanoemulsions after application onto mucosa or contact with saliva or other water media, demonstrated fast and efficient protection against PTZ-induced seizures when administered via intraoral transmucosal route. Combination of eutectic mixture nanoemulsion vehicle with volatile organic solvents helps to prepare sprayable formulations, suitable for intraoral buccal or sublingual delivery and provides fast and effective anti-seizure action.

Graph 1. PTZ induced seizures protection by marketed injectable formulation of Lorazepam (2.5 mg/kg), given parenterally and sublingually, compared to the vehicle

Graph 2. PTZ induced seizures protection by different transmucosal formulations of Lorazepam (2.5 mg/kg) with or without Menthol

Ex. 1 - 2% of peppermint oil (-40% Menthol content) based formulation; forms micellar solution

Ex. 2 - 2.4% of crystalline L-Menthol, no peppermint oil; self-nanoemulsifying formulation

Ex. 3 - Does not contain Menthol or Peppermint oil; self-nanoemulsifying formulation

Graph 3. PTZ induced seizures protection by transmucosal formulations of Lorazepam (2.5 mg/kg), administered sublingually 2 and 5 minutes before PTZ infusion initiation

Graph 4. Particle size and size distribution for nanoemulsion of Example 18

Hydrodynamic diameter (Z-Average) - 44.7 nm
Particle size distribution: Mean diameter (volume) - 40.8 nm $\pm$ 11.9 nm
Polydispersity index 0.150

**References:**

**Patents and patent applications**

U.S. Patent No. 6,627,211 B1

US Patent Application 20050002987 A1

Clonazepam spray US Patent Application US20080070904

Intranasal benzodiazepine spray US Patent Application US20110172211A1

Giovannone D. et al. Liquid Compositions for Oral Administration of Lorazepam" WO 2004/004783 A1

**Articles**

Behrouz R., Chen S.,. Tatum W. O. Evaluation and Management of Status Epilepticus in the Neurological Intensive Care Unit. J Am Osteopath Assoc. 2009, 109(4), pp.237-245.

Tatum W. O. IV, French J. A., Benbadis S. R., Kaplan P. W. The Etiology and Diagnosis of Status Epilepticus. Epilepsy Behav. 2001, 2(4), pp.311-317.

Wermeling D. P. Intranasal Delivery of Antiepileptic Medications for Treatment of Seizures. Neurotherapeutics, 2009, 6(2), pp.352-358.

Anderson G.D., Saneto R.P. Current oral and non-oral routes of antiepileptic drug delivery. Adv. Drug Deliv. Rev. 2012, 64(10), pp.911-8.

Jevon P. Buccolam® (buccal midazolam): a review of its use for the treatment of prolonged acute convulsive seizures in the dental practice. British Dental Journal, 2012, 213, pp.81 - 82.

Lau S.W.J., Slattery J.T. Absorption of diazepam and lorazepam following intranasal administration. Int. J. Pharm. 1989, 54(2), pp.171-174.

Li L., Nandi I., Kim K.H. Development of an ethyl laurate-based microemulsion for rapid-onset intranasal delivery of diazepam. Int J Pharm. 2002, 237(1-2), pp.77-85. Hammad M. "Solubility and Stability of Lorazepam in Bile Salt/Soya Phosphatidylcholine - Mixed Micelles", Drug Development and Industrial Pharmacy, 25(4), 409-417 (1999)

Giardina WJ, Gasior M., 2009. "Acute seizure tests in epilepsy research: electroshock- and chemical- induced convulsions in the mouse". Current Protocols in Pharmacology, 45, 5.22.1-5.22.37.

Mandhane, S.N., Aavula, K., Rajamannar, T., 2007. "Timed pentylenetetrazol infusion test: a comparative analysis with s.c. PTZ and MES models of anticonvulsant screening in mice". Seizure, 16, 636-644.

**Claims**

1. A liquid pharmaceutical composition for intraoral transmucosal administration of a benzodiazepine drug to a mammal in need, the composition comprising: a physiologically acceptable hydrophobic phase; an eutectic mixture of benzodiazepine compound providing high solubility of the benzodiazepine in said hydrophobic phase; and at least one physiologically acceptable surfactant; wherein said benzodiazepine is Lorazepam.

2. The composition of claim 1, wherein said eutectic mixture comprises said Lorazepam and a cyclic alcohol.

3. The composition of claim 1, wherein said eutectic mixture comprises said Lorazepam and a phenolic substance.

4. The composition of claim 2, wherein said cyclic alcohol is selected from a group consisting of D-Menthol, L-Menthol, rac-Menthol, Neo-Menthol, iso-Menthol, Cyclohexanol, Borneol, iso-Borneol, or mixture thereof.

5. The composition of claim 3, wherein said phenolic substance is selected from a group consisting of Phenol, Thymol, Resorcinol, Carvacrol, Butylated Hydroxytoluene, Butylated Hydroxyanisole, 2,6-Diisopropyl-Phenol, p-Cresol, m-Cresol, or mixture thereof.

6. The composition of claim 4, wherein said cyclic alcohol is L-Menthol.

7. The composition of claim 5, wherein said phenolic substance is Thymol.

8. The composition of claim 6, wherein said Lorazepam and said L-Menthol are in molar ratio from 1:10 to 10:1, preferably 1:5 to 5:1.

9. The composition of claim 1, wherein said composition contains from 0.01 to 10% of Menthol.

10. The composition of claim 1, wherein said composition can be delivered intraorally using a dropper, a dose pump, or a metered dose device.

11. The composition of claim 1, wherein said composition forms micelles after contact with saliva or aqueous media.

12. The composition of claim 1, wherein said composition forms oil-in-water emulsion after contact with saliva or aqueous media.

13. The composition of claim 1, wherein lorazepam is completely dissolved in the composition at a concentration from 1 to 100 mg/mL and does not precipitates or forms crystals after contact with saliva or other biological media.

14. The composition of claim 1, wherein said hydrophobic phase comprises ef aliphatic or aromatic esters, mono-, di- or triglycerides, tocopheryl esters, acetylated glycerides, edible essential oils, or mixture thereof.


**Patentansprüche**

1. Flüssige pharmazeutische Zusammensetzung zur intraoralen transmukosalen Verabreichung eines Benzodiazepinarzneimittels an ein Säugetiers, das dies benötigt, wobei die Zusammensetzung umfasst: eine physiologisch unbedenkliche hydrophobe Phase; ein eutektisches Gemisch aus Benzodiazepinverbindung, die eine hohe Löslichkeit des Benzodiazepins in der hydrophoben Phase bereitstellt; und zumindest ein physiologisch unbedenkliches Tensid; wobei das Benzodiazepin Lorazepam ist.

2. Zusammensetzung nach Anspruch 1, wobei das eutektische Gemisch das Lorazepam und einen cyclischen Alkohol umfasst.

3. Zusammensetzung nach Anspruch 1, wobei das eutektische Gemisch das Lorazepam und eine phenolische Substanz umfasst.

4. Zusammensetzung nach Anspruch 2, wobei der cyclische Alkohol aus einer Gruppe ausgewählt ist, die aus D-Menthol, L-Menthol, rac-Menthol, Neo-Menthol, iso-Menthol, Cyclohexanol, Borneol, iso-Borneol oder einem Gemisch davon besteht.

5. Zusammensetzung nach Anspruch 3, wobei die phenolische Substanz aus einer Gruppe ausgewählt ist, die aus Phenol, Thymol, Resorcinol, Carvacrol, butyliertem Hydroxytoluol, butyliertem Hydroxyanisol, 2,6-Diisopropylphenol, p-Cresol, m-Cresol oder einem Gemisch davon besteht.

6. Zusammensetzung nach Anspruch 4, wobei der cyclische Alkohol L-Menthol ist.

7. Zusammensetzung nach Anspruch 5, wobei die phenolische Substanz Thymol ist.

8. Zusammensetzung nach Anspruch 6, wobei das Lorazepam und das L-Menthol in einem Molverhältnis von 1:10 bis 10:1, vorzugsweise 1:5 bis 5:1, vorliegen.

9. Zusammensetzung nach Anspruch 1, wobei die Zusammensetzung 0,01 bis 10 % Menthol enthält.

10. Zusammensetzung nach Anspruch 1, wobei die Zusammensetzung intraoral unter Verwendung einer Pipette, einer Dosierpumpe oder einer Dosiervorrichtung abgegeben werden kann.

11. Zusammensetzung nach Anspruch 1, wobei die Zusammensetzung nach Kontakt mit Speichel oder wässrigem Medium Mizellen bildet.

12. Zusammensetzung nach Anspruch 1, wobei die Zusammensetzung nach Kontakt mit Speichel oder wässrigem Medium eine Öl-in-Wasser-Emulsion bildet.

13. Zusammensetzung nach Anspruch 1, wobei Lorazepam in der Zusammensetzung in einer Konzentration von 1 bis 100 mg/ml vollständig gelöst ist und nach Kontakt mit Speichel oder anderen biologischen Medien nicht ausfällt oder Kristalle bildet.

14. Zusammensetzung nach Anspruch 1, wobei die hydrophobe Phase aliphatische oder aromatische Ester, Mono-, Di- oder Triglyzeride, Tocopherylester, acetylierte Glyzeride, essbare essentielle Öle oder Gemische davon umfasst.


**Revendications**

1. Composition pharmaceutique liquide pour administration intra-orale par voie trans-muqueuse d'un médicament benzodiazépine à un mammifère en ayant besoin, la composition comprenant : une phase hydrophobe physiologiquement acceptable ; un mélange eutectique de composé benzodiazépine assurant une solubilité élevée de la benzodiazépine dans ladite phase hydrophobe ; et au moins un tensioactif physiologiquement acceptable ; dans laquelle ladite benzodiazépine est le lorazépam.

2. Composition selon la revendication 1, dans laquelle ledit mélange eutectique comprend ledit lorazépam et un alcool cyclique.

3. Composition selon la revendication 1, dans laquelle ledit mélange eutectique comprend ledit lorazépam et une substance phénolique.

4. Composition selon la revendication 2, dans laquelle ledit alcool cyclique est choisi dans le groupe constitué par le D-menthol, le L-menthol, le rac-menthol, le néo- menthol, l'iso-menthol, le cyclohexanol, le bornéol, l'iso-bornéol, et leurs mélanges.

5. Composition selon la revendication 3, dans laquelle ladite substance phénolique est choisie dans le groupe constitué par le phénol, le thymol, le résorcinol, le carvacrol, l'hydroxytoluène butylé, l'hydroxyanisole butylé, le 2,6-diisopropylphénol, le p-crésol, le m-crésol, et leurs mélanges.

6. Composition selon la revendication 4, dans laquelle ledit alcool cyclique est le L-menthol.

7. Composition selon la revendication 5, dans laquelle ladite substance phénolique est le thymol.

8. Composition selon la revendication 6, dans laquelle ledit lorazépam et ledit L-menthol sont présents en un rapport molaire de 1/10 à 10/1, de préférence de 1/5 à 5/1.

9. Composition selon la revendication 1, laquelle composition contient de 0,01 à 10 % de menthol.

10. Composition selon la revendication 1, laquelle composition peut être délivrée par voie intra-orale par utilisation d'un compte-gouttes, d'une pompe doseuse, ou d'un dispositif doseur.

11. Composition selon la revendication 1, laquelle composition forme des micelles après contact avec la salive ou un milieu aqueux.

12. Composition selon la revendication 1, laquelle composition forme une émulsion huile dans l'eau après contact avec la salive ou un milieu aqueux.

13. Composition selon la revendication 1, dans laquelle le lorazépam est complètement dissous dans la composition à une concentration de 1 à 100 mg/ml et ne précipite pas ou ne forme pas de cristaux après contact avec la salive ou un autre milieu biologique.

14. Composition selon la revendication 1, dans laquelle ladite phase hydrophobe comprend des esters aliphatiques ou aromatiques, des mono-, di- ou tri-glycérides, des esters de tocophéryle, des glycérides acétylés, des huiles essentielles comestibles, ou leurs mélanges.

PTZ induced seizures protection by marketed injectable formulation of Lorazepam (2.5 mg/kg)
given parenterally and sublingually, compared to vehicle

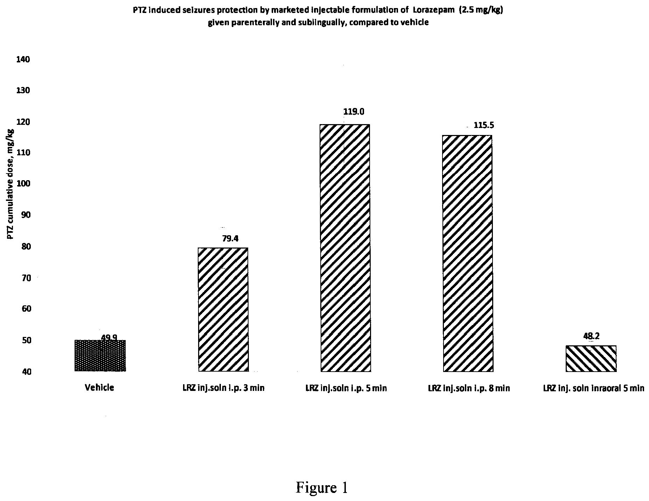

Figure 1

PTZ induced seizures protection by transmucosal formulations of Lorazepam (2.5 mg/kg)

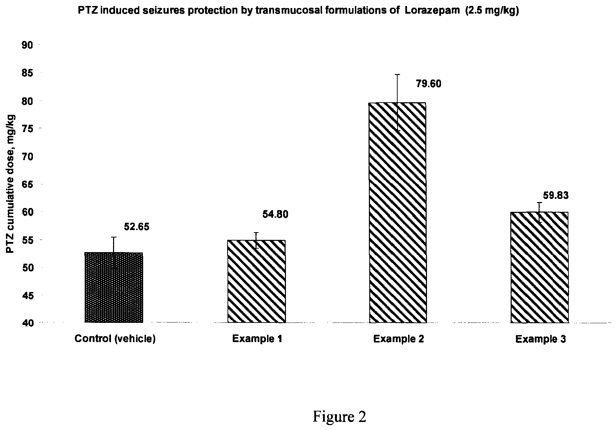

Figure 2

PTZ induced seizures protection by transmucosal formulations of Lorazepam (2.5 mg/kg),
administered sublingually 2 and 5 minutes before PTZ infusion start

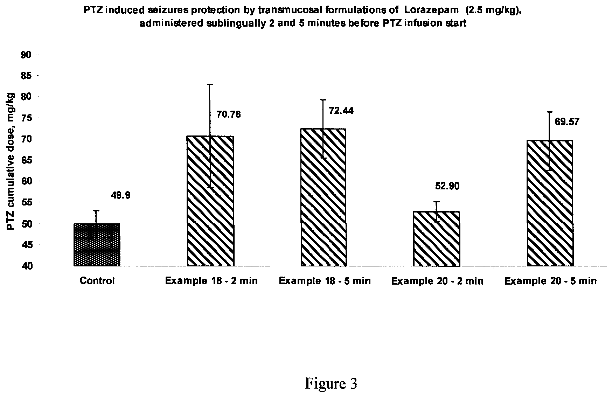

Figure 3

Size Distribution by Volume

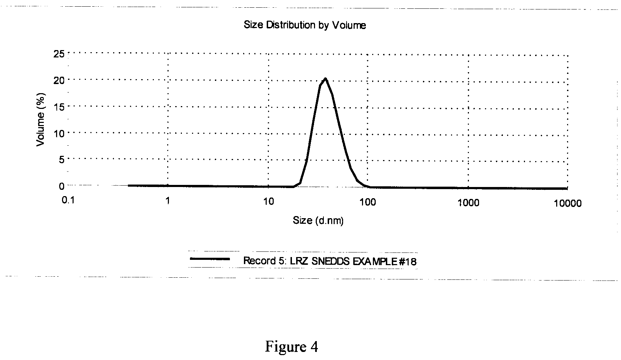

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20110172211 A
- US 6627211 B1
- US 20050002987 A1
- US 20080070904 A
- US 20110172211 A1
- WO 2004004783 A1
- WO 2011135314 A

### Non-patent literature cited in the description

- **BEHROUZ R. ; CHEN S. ; TATUM W. O.** Evaluation and Management of Status Epilepticus in the Neurological Intensive Care Unit. *J Am Osteopath Assoc.,* 2009, vol. 109 (4), 237-245
- **TATUM W. O. IV ; FRENCH J. A. ; BENBADIS S. R. ; KAPLAN P. W.** The Etiology and Diagnosis of Status Epilepticus. *Epilepsy Behav,* 2001, vol. 2 (4), 311-317
- **WERMELING D. P.** Intranasal Delivery of Antiepileptic Medications for Treatment of Seizures. *Neurotherapeutics,* 2009, vol. 6 (2), 352-358
- **ANDERSON G.D. ; SANETO R.P.** Current oral and non-oral routes of antiepileptic drug delivery. *Adv. Drug Deliv. Rev.,* 2012, vol. 64 (10), 911-8
- **JEVON P.** Buccolam® (buccal midazolam): a review of its use for the treatment of prolonged acute convulsive seizures in the dental practice. *British Dental Journal,* 2012, vol. 213, 81-82
- **LAU S.W.J. ; SLATTERY J.T.** Absorption of diazepam and lorazepam following intranasal administration. *Int. J. Pharm.,* 1989, vol. 54 (2), 171-174
- **LI L. ; NANDI I. ; KIM K.H.** Development of an ethyl laurate-based microemulsion for rapid-onset intranasal delivery of diazepam. *Int J Pharm.,* 2002, vol. 237 (1-2), 77-85
- **HAMMAD M.** Solubility and Stability of Lorazepam in Bile Salt/Soya Phosphatidylcholine - Mixed Micelles. *Drug Development and Industrial Pharmacy,* 1999, vol. 25 (4), 409-417
- **GIARDINA WJ ; GASIOR M.** Acute seizure tests in epilepsy research: electroshock- and chemical- induced convulsions in the mouse. *Current Protocols in Pharmacology,* 2009, vol. 45, 5.22.1-5.22.37
- **MANDHANE, S.N. ; AAVULA, K. ; RAJAMANNAR, T.** Timed pentylenetetrazol infusion test: a comparative analysis with s.c. PTZ and MES models of anticonvulsant screening in mice. *Seizure,* 2007, vol. 16, 636-644